# EUROPEAN PATENT SPECIFICATION

(11) **EP 2 583 718 B1**
(45) Date of publication and mention of the grant of the patent: **10.01.2018**
(21) Application number: 10853300.1
(22) Date of filing: 17.11.2010
(51) Int. Cl.: A61N 5/06, A61H 19/00

(54) **PORTABLE DEVICE FOR USE IN TREATMENT OF URINARY INCONTINENCE AND DYSMENORRHEA USING PHOTON ENERGY**
TRAGBARE VORRICHTUNG ZUR BEHANDLUNG VON HARNINKONTINENZ UND DYSMENORRHOE MIT PHOTONENENERGIE
DISPOSITIF PORTATIF DESTINÉ AU TRAITEMENT DE L'INCONTINENCE URINAIRE ET DE LA DYSMÉNORRHÉE AU MOYEN DE L'ÉNERGIE PHOTONIQUE

(30) Priority: 16.06.2010 KR 20100057078
(43) Date of publication of application: 24.04.2013
(73) Proprietor: Color Seven.Co., Ltd, Seoul 06719 (KR)
(72) Inventor: KIM, Nam Gyun, Jeonju-si Jeollabuk-do 560-793 (KR); PARK, Kyoung Jun, Seoul 135-270 (KR)
(74) Representative: Grünecker Patent- und Rechtsanwälte PartG mbB
(86) International application number: PCT/KR2010/008128
(87) International publication number: WO 2011/158999

(56) References cited:
- DE-U1- 20 214 963
- JP-A- 11 267 178
- KR-Y1- 200 233 009
- KR-Y1- 200 404 899
- US-A1- 2006 167 531
- US-A1- 2007 162 092
- US-A1- 2008 119 831

## Description

### BACKGROUND

### 1. Field of the Invention

The present invention relates to a device for use in treatment of urinary incontinence and dysmenorrhea, and more particularly, to a portable device for use in treatment of urinary incontinence and dysmenorrhea using photon energy.

### 2. Discussion of Related Art

Urinary incontinence is a disease causing involuntary leakage of urine when urination is not desired, for example, when laughing, coughing, or running, which may cause sanitary problems, serious reduction of activity, and depression.

As a treatment device for treating such urinary incontinence, a urinary incontinence treatment device disclosed in Korean Patent Registration No. 10-0824014 (Registration Date: April 15, 2008) has been developed. The urinary incontinence treatment device is inserted into a human body to treat the urinary incontinence, and includes a holder disposed under the urethra in the body and configured to hold the urethra, and a support supported in the body and configured to support the holder to maintain the holder in the body at a desired position.

In addition, as a treatment device for treating such urinary incontinence, a capsule-type urinary incontinence treatment device disclosed in Korean Patent Registration No. 10-0684927 (Registration Date: February 13, 2007) has been developed. The urinary incontinence treatment device is configured to seal a capsule having a space formed by an upper cap and a lower cap, bions generated by anions and far-infrared light fill the capsule, and a hook is formed at an upper end of the capsule and a string is bound to the hook.

Meanwhile, dysmenorrhea is a disease caused by excessive worrying or severe stress. In general, while pains of the lower abdomen and the waist when a woman menstruates are referred to as a dysmenorrhea, the dysmenorrhea may be accompanied with indigestion, vomiting, diarrhea, headache, depression and mental tension, in addition to backaches or pantalgia.

As a device for use in treatment of dysmenorrhea, a device for use in treatment of dysmenorrhea disclosed in Korean Patent Application Laid-Open No. 10-2001-0019454 (Publication Date: March 15, 2001) had been developed. The device for use in treatment of dysmenorrhea is inserted into the uterus to spread the uterus such that blood circulation becomes normal to effectively suppress the dysmenorrhea.

KR 20-0404899 Y1 discloses the use of a laser diode for improving blood circulation, wherein the laser diode emits light in the red to infrared wavelength range.

### SUMMARY OF THE INVENTION

However, since both of the above-mentioned conventional urinary incontinence treatment device and the dysmenorrhea treatment device are inserted into the vagina, the treatment device is inconvenient in use and has sanitary disadvantages.

In order to solve these problems, the present invention provides a portable device for use in treatment of urinary incontinence and dysmenorrheal using photon energy that can be hung by string to be used asa necklace or won on the waist. A color electrode wire to which at least one color electrode including an LED configured to emit colored light (for example, orange, red, green, yellow or the like) having a predetermined wavelength range (for example, 400 nm to 850 nm) for treatment of urinary incontinence or dysmenorrhea is connected to a main body and used. The color electrode is directly attached to a urinary incontinence treatment area or a dysmenorrhea treatment area of the body related to the uterus, the bladder, and the kidney, for example, a meridian point in oriental medicine such as the *Gihae* meridian point which functions to circulate and activate the whole body to discharge carbon dioxide and attenuate all pains beneath the belly button, the *Junggeuk* meridian point which maintains a balance between storage and discharge of urine, discharges the wet, and maintains harmony of blood and the nervous system, the *Sudo* meridian point which classifies and harmonizes moisture, evenly divides the moisture and solid at a certain ratio, and attenuates nerve stimuli, and the *Jung-wan* meridian point which stimulates activity of the brain to make good energy and generate energy to activate metabolism. The colored light is radiated for a predetermined time (for example, 10±5 minutes) to supply photon energy to damaged cells, activating metabolism of the cells, promoting blood circulation, and treating the urinary incontinence and dysmenorrhea.

In order to accomplish the object, the present invention is directed to a portable device for use in treatment of urinary incontinence and dysmenorrhea using photon energy, comprising: a case having a hollow tubular shape; a treatment mode selection switch installed at one surface of the case and configured to output a treatment mode selection signal of either of a urinary incontinence treatment mode and a dysmenorrhea treatment mode; a treatment operation button installed at one surface of the case and configured to output a treatment operation signal whenever the button is pushed one time; a color electrode wire having one end to which at least one color electrode comprising a light emitting diode (LED) configured to emit colored light having a predetermined wavelength range for urinary incontinence treatment or dysmenorrhea treatment is connected, and the other end to which an electrode pin is connected; at least one electrode jack installed at one surface of the case and inserted and connected to the electrode pin of the color electrode wire; a microprocessor installed in the case, configured to output a treatment start signal of either of urinary incontinence treatment and dysmenorrhea treatment whenever a treatment operation signal is input after a treatment mode selection signal of either of the urinary incontinence treatment mode and the dysmenorrhea treatment mode is input, and configured to output a treatment termination signal when a predetermined time elapses after the treatment start signal is output; a treatment signal generator configured to output a treatment signal to emit colored light having a predetermined wavelength range for urinary incontinence treatment or dysmenorrhea treatment when a treatment start signal of either of the urinary incontinence treatment and the dysmenorrhea treatment to turn on the LED of the color electrode is input, and stop output of the treatment signal when the treatment termination signal is input; and a power supply unit configured to supply driving power of the treatment mode selection switch, the treatment operation button, the color electrode, the microprocessor, and the treatment signal generator.

In the portable device for use in treatment of urinary incontinence and dysmenorrhea using photon energy according to the present invention, the power supply unit may be a disposable battery or rechargeable battery configured to supply the driving power of the treatment mode selection switch, the treatment operation button, the color electrode, the microprocessor, and the treatment signal generator, and the rechargeable battery may receive charge power through a charge adapter inserted and connected to a charge jack installed at one surface of the case.

In the portable device for use in treatment of urinary incontinence and dysmenorrhea using photon energy according to the present invention, the color electrode may include: the LED configured to emit colored light having a predetermined wavelength range for urinary incontinence treatment or dysmenorrhea treatment; an optical filter configured to filter, amplify and collect the colored light emitted from the LED; an electrode case having a hollow tubular shape and comprising a rear surface to which the color electrode wire is connected, an inner space in which the LED and the optical filter are housed, and a front surface having a hole through which the colored light passing through the optical filter is discharged to the outside, wherein one surface of a double-sided sticker is attached to a periphery of the hole of the front surface, and the other surface of the sticker is attached to a urinary incontinence treatment area or a dysmenorrhea treatment area of the body.

In the portable device for use in treatment of urinary incontinence and dysmenorrhea using photon energy according to the present invention, the optical filter may be formed of any one of quartz, crystal, and crystal glass, and has a transmission surface cut in a polygonal shape or a convex lens shape such that the colored light emitted from the LED is intensively radiated to a urinary incontinence treatment area or a dysmenorrhea treatment area of the body through the hole of the electrode case.

The portable device for use in treatment of urinary incontinence and dysmenorrhea using photon energy according to the present invention may further includes a speaker configured to inform the user of treatment termination through an alarm sound or voice, or a vibrator configured to inform the user of treatment termination through vibrations when the microprocessor outputs a treatment termination signal.

In the portable device for use in treatment of urinary incontinence and dysmenorrhea using photon energy according to the present invention, a loop to which a string is able to be bound to use the case like a necklace, or a clip that enables the case to be worn on a waist belt, may be formed at one surface of the case.

In the portable device for use in treatment of urinary incontinence and dysmenorrhea using photon energy according to the present invention, the microprocessor may be in wireless communication with a wireless treatment mode selector configured to output a treatment mode selection signal of either of a urinary incontinence treatment mode and a dysmenorrhea treatment mode through a wireless communication unit in a wireless manner to receive a treatment mode selection signal.

In the portable device for use in treatment of urinary incontinence and dysmenorrhea using photon energy according to the present invention, the wireless treatment mode selector may be any one of a personal computer (PC), a notebook, a smart phone, and a personal digital assistant (PDA), in which a program in wireless communication with the wireless communication unit of the microprocessor to transmit a treatment mode selection signal of either of the urinary incontinence treatment mode and the dysmenorrhea treatment mode through any one of radio frequency (RF), Bluetooth, and ZigBee types is installed, and providing a user interface (UI) screen that is able to transmit a treatment mode selection signal.

### BRIEF DESCRIPTION OF THE DRAWINGS

The above and other objects, features and advantages of the present invention will become more apparent to those of ordinary skill in the art by describing in detail example embodiments thereof with reference to the attached drawings, in which:
FIG. 1 is a perspective view of a portable device for use in treatment of urinary incontinence and dysmenorrhea using photon energy according to the present invention;
FIG. 2 is a block diagram of the portable device for use in treatment of urinary incontinence and dysmenorrhea using photon energy according to the present invention; and
FIG. 3 is a view showing an embodiment of a use state of the portable device for use in treatment of urinary incontinence and dysmenorrhea using photon energy according to the present invention.

### DETAILED DESCRIPTION OF EXAMPLE EMBODIMENTS

Hereinafter, an example embodiment of the present invention will be described in detail with reference to the accompanying drawings.

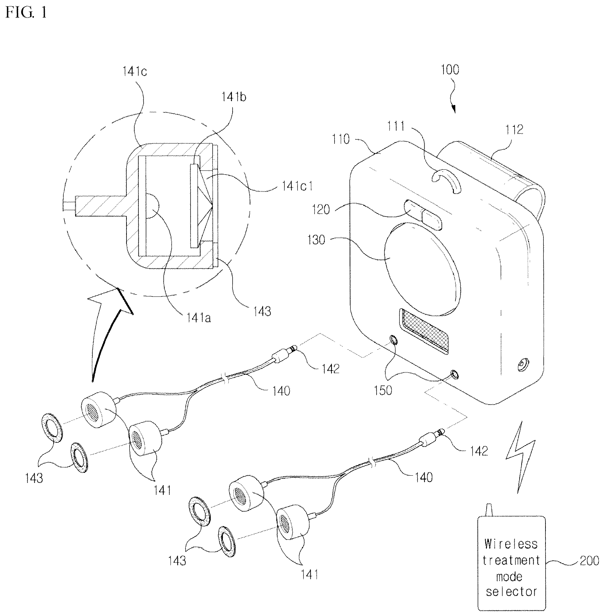

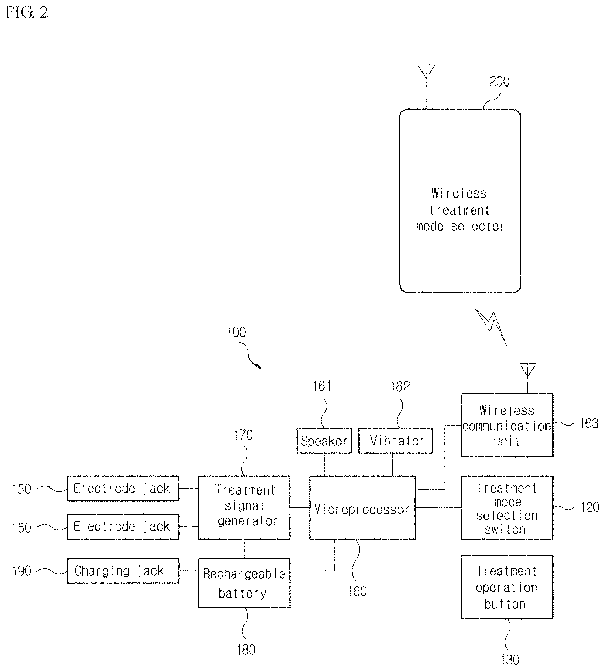

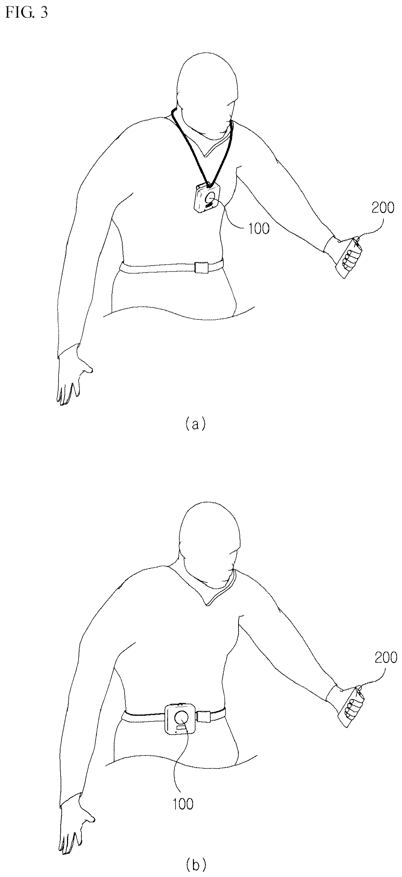

Referring to FIGS. 1 to 3, a case 110 has a hollow tubular shape. While FIGS. 1 and 3 exemplify the case having a rectangular parallelepiped shape, the case 110 may be modified into various shapes such as a flat cylindrical shape, an oval tube shape, or a heart-shaped tube.

A loop 111 to which a string can be bound to use the case 110 like a necklace or a clip 112 which enables wearing of the case 110 on a waist belt is formed at one surface of the case 110.

The loop 111 may be formed at, for example, an upper surface of the case 110 shown in FIG. 1, and the clip 112 may be formed at, for example, a rear surface of the case 110 shown in FIG. 1.

As shown in FIG. 3 (a) and (b), the case 110 may be used as a necklace or may be worn on a waist belt.

A treatment mode selection switch 120 is installed at one surface of the case 110, for example, a front surface of the case 110 shown in FIG 1, and outputs a treatment mode selection signal of either of a urinary incontinence treatment mode and a dysmenorrhea treatment mode.

A treatment operation button 130 is installed at one surface of the case 110, for example, the front surface of the case 110 shown in FIG. 1, and outputs a treatment operation signal whenever the button is pushed one time.

At least one color electrode 141 including an LED 141a configured to emit colored light (for example, orange, red, green, yellow or the like) having a predetermined wavelength range (for example, 400 nm to 850 nm) for urinary incontinence treatment or dysmenorrhea treatment is connected to one end of a color electrode wire 140, and an electrode pin 142 is connected to the other end thereof.

As shown by a partially enlarged view of FIG. 1, the LED 141a of the color electrode 141, which may be a single color LED, a three-color LED, or the like, emits colored light having a predetermined wavelength range for urinary incontinence treatment or dysmenorrhea treatment.

An optical filter 141b of the color electrode 141 filters, amplifies and collects the colored light emitted from the LED 141a.

The optical filter 141 b may be formed of quartz, crystal, crystal glass, or the like. In particular, a transmission surface of the filter is cut into a polygonal (for example, pentagonal, hexagonal, octagonal, or the like) shape or formed in a convex lens shape such that the colored light emitted from the LED 141a is intensively radiated to a urinary incontinence treatment area of dysmenorrhea treatment of the body through a hole 141c1 of an electrode case 141c.

The electrode case 141c of the color electrode 141 has a hollow tubular shape, and includes a rear surface connected to the color electrode wire 140, an inner space in which the LED 141a and the optical filter 141b are housed, and a front surface having the hole 141c1 through which the colored light passing through the optical filter 141b is discharged to the outside.

One surface of a double-sided sticker 143 is attached to a periphery of the hole 141c1 of the front surface of the electrode case 141c, and the other surface of the sticker 143 is attached to a urinary incontinence treatment area or a dysmenorrhea treatment area of the body. For reference, the urinary incontinence treatment area or the dysmenorrhea treatment area of the body is a meridian point of the body related to the uterus, the bladder, or the kidney in oriental medicine, for example, the *Gihae* meridian point which functions to circulate and activate the whole body to discharge carbon dioxide and attenuate all pains beneath the belly button, the *Junggeuk* meridian point which maintains a balance between storage and discharge of urine, discharges the wet, and maintains harmony of blood and the nervous system, the *Sudo* meridian point which classifies and harmonizes moisture, evenly divides the moisture and solid at a certain ratio, and attenuates nerve stimuli, and the *Jung-wan* meridian point which stimulates activity of the brain to make good energy and generate energy to activate metabolism.

At least one electrode jack 150 is installed at one surface of the case 110, for example, the front surface of the case 110 shown in FIG. 1, and the electrode pin 142 of the color electrode wire 140 is inserted thereinto.

A microprocessor 160 is installed in the case 110, and whenever a treatment operation signal is input after a treatment mode selection signal of either of the urinary incontinence treatment mode and the dysmenorrhea treatment mode is input, a treatment start signal of either of the urinary incontinence treatment and the dysmenorrhea treatment is output. When a predetermined time (for example, 10±5 minutes) elapses after output of the treatment start signal, a treatment termination signal is output.

The microprocessor 160 outputs the treatment termination signal to inform a user of treatment termination through a speaker 161 configured to generate an alarm sound or voice, or inform the user of the treatment termination through a vibrator 162 configured to generate vibrations.

The microprocessor 160 is in wireless communication with a wireless treatment mode selector 200 configured to output a treatment mode selection signal of either of the urinary incontinence treatment mode and the dysmenorrhea treatment mode in a wireless manner through a wireless communication unit 163 using a driving power of a disposable battery or a rechargeable battery 180 to receive a treatment mode selection signal.

The wireless treatment mode selector 200 can be held in a user's hand as shown in FIGS. 3(a) and 3(b).

The wireless treatment mode selector 200 is a portable wireless communication device such as a personal computer (PC), a notebook, a smart phone, a personal digital assistant (PDA), or the like, in which a program in communication with the wireless communication unit 163 of the microprocessor 160 through various wireless communication types such as a radio frequency (RF) type, Bluetooth, ZigBee, and so on, in a wireless communication manner is installed to transmit a treatment mode selection signal of either of the urinary incontinence treatment mode and the dysmenorrhea treatment mode, providing a user interface (UI) screen that can transmit a treatment mode selection signal.

The program than can transmit a treatment mode selection signal of either of the urinary incontinence treatment mode and the dysmenorrhea treatment mode installed in the PC, notebook, smart phone, PDA and so on, and the UI screen that can transmit a treatment mode selection signal can be easily understood and variously implemented by those skilled in the art. For example, when the user selects a icon of the device for use in treatment of urinary incontinence and dysmenorrhea installed on a desktop of the PC or notebook, or a widget screen of the smart phone or PDA, the program configured to provide the UI screen that can transmit the corresponding treatment mode selection signal to the outside after either of the urinary incontinence treatment mode and the dysmenorrhea treatment mode is selected can be implemented.

The speaker 161 may be installed at one surface of the case 110, for example, the front surface, a side surface or the rear surface of the case 110 shown in FIG. 1.

The vibrator 162 may be installed in the case 110.

A treatment signal generator 170 outputs a treatment signal of emitting colored light having a predetermined wavelength range for urinary incontinence treatment or dysmenorrhea treatment when a treatment start signal of either of the urinary incontinence treatment and the dysmenorrhea treatment is input to turn on the LED 141a of the color electrode 141, and stops output of the treatment signal when a treatment termination signal is input.

The treatment signal generator 170 outputs a treatment signal to emit a single color of light or sequentially emit a plurality of colors of light when the treatment start signal is input.

For example, when the treatment signal generator 170 outputs the treatment signal to emit the single color of light, the LED 141a of the color electrode 141 emits the single color of light such as orange, red, green, yellow, or the like. Here, when a plurality of color electrodes 141 are connected to at least one electrode jack 150, all of the color electrodes 141 may emit the same single color of light, or may emit different single colors of light.

Unlike this, when the treatment signal generator 170 outputs a treatment signal to sequentially emit a plurality of colors of light, the LED 141a of the color electrode 141 emits three colors of light including orange, red, green, and yellow, or two colors of light in a predetermined sequence, for example, in a sequence of orange → red → green → yellow, or orange → red.

A power supply unit supplies driving power of the treatment mode selection switch 120, the treatment operation button 130, the color electrode 141, the microprocessor 160, and the treatment signal generator 170.

The power supply unit is a disposable battery or a rechargeable battery 180 configured to supply driving power of the treatment mode selection switch 120, the treatment operation button 130, the color electrode 141, the microprocessor 160, and the treatment signal generator 170. The rechargeable battery 180 receives charge power through a charge adapter inserted into and connected to a charge jack 90 installed at one surface of the case 110, for example, a side surface of the case 110 shown in FIG. 1, when the power is consumed. The charge adapter may be connected to a conventional AC power source to generate a constant voltage of 12 V, and the charge adapter will be readily understood and implemented by those skilled in the art without specific illustration and description.

The portable device for use in treatment of urinary incontinence and dysmenorrhea using photon energy according to the present invention configured as above will be used as follows.

The user first inserts and connects the electrode pin 142 of the color electrode wire 140, to which the at least one color electrode 141 is connected, to the at least one electrode jack 150.

Next, the user attaches one surface of the double-sided sticker 143 to a periphery of the hole 141c1 of the front surface of the electrode case 141c of the color electrode 141, and then attaches the other surface of the sticker 143 to the urinary incontinence treatment area or the dysmenorrhea treatment area of the body.

In a state in which the color electrode 141 is attached to the urinary incontinence treatment area or the dysmenorrhea treatment area of the body by the double-sided sticker 143, the user first manipulates the treatment mode selection switch 120 to output a treatment mode selection signal of either of the urinary incontinence treatment mode and the dysmenorrhea treatment mode, or manipulates the wireless treatment mode selector 200 to output a treatment mode selection signal of either of the urinary incontinence treatment mode and the dysmenorrhea treatment mode in a wireless manner.

The output treatment mode selection signal of either of the urinary incontinence treatment mode and the dysmenorrhea treatment mode is directly input into the microprocessor 160 or input into the microprocessor 160 via the wireless communication unit 163, and then the treatment operation signal is output to be input into the microprocessor 160 whenever the user pushes the treatment operation button 130.

Accordingly, the microprocessor 160 outputs a treatment start signal of either of the urinary incontinence treatment and the dysmenorrhea treatment to transmit the signal to the treatment signal generator 170 whenever the treatment operation signal is input, and then the treatment signal generator 170 outputs a treatment signal to emit colored light (for example, orange, red, green, yellow or the like) having a predetermined wavelength range (for example, 400 nm to 850 nm) for the urinary incontinence treatment or the dysmenorrhea treatment to turn on the LED 141a of the color electrode 141. Accordingly, the colored light emitted from the LED 141a of the color electrode 141 is filtered, amplified and collected by the optical filter 141b, and then intensively radiated to the urinary incontinence treatment area or the dysmenorrhea treatment area through the hole 141c1 of the electrode case 141c.

When a predetermined time (for example, 10±5 minutes) elapses after the treatment start signal of the microprocessor 160 is output, the microprocessor 160 outputs a treatment termination signal.

Here, the speaker 161 generates an alarm sound or voice, and the vibrator 162 generates vibrations, informing the user of treatment termination.

In addition, the treatment signal generator 170 stops output of the treatment signal when the treatment termination signal is input, and at this time, the LED 141a of the color electrode 141 is turned off.

In this state, when the user intends to continue the treatment using the colored light, the user pushes the treatment operation button 130 once more, and the treatment using the colored light is repeated whenever the user pushes the treatment operation button 130 one time.

As can be seen from the foregoing, since the color electrode is attached to the urinary incontinence treatment area or the dysmenorrhea treatment area of the body to intensively radiate the colored light, an unsanitary problem of the conventional urinary incontinence treatment device or the dysmenorrhea treatment device inserted into the vagina can be completely solved and urinary incontinence and dysmenorrhea treatment effects can be expected. In addition, since the device can be used like a necklace or can be worn on a waist belt, the device can be easily carried and easily used regardless of use places.

While the portable device for use in treatment of urinary incontinence and dysmenorrhea using photon energy of the invention, which is not limited to the above-mentioned embodiment, has been shown and described with reference to certain example embodiments thereof, it will be understood by those skilled in the art that various changes in form and details may be made therein without departing from the scope of the invention as defined by the appended claims.

## Claims

1. A portable device for use in treatment of urinary incontinence and dysmenorrhea using photon energy, comprising:
a case (110) having a hollow tubular shape;
a treatment mode selection switch (120) installed at one surface of the case (110) and configured to output a treatment mode selection signal of either of a urinary incontinence treatment mode and a dysmenorrhea treatment mode;
a treatment operation button (130) installed at one surface of the case (110) and configured to output a treatment operation signal whenever the button is pushed one time;
a color electrode wire (140) having one end to which at least one color electrode (141) comprising a light emitting diode (LED) (141a) configured to emit colored light having a predetermined wavelength range for urinary incontinence treatment or dysmenorrhea treatment is connected, and another end to which an electrode pin (142) is connected;
at least one electrode jack (150) installed at one surface of the case (110), to which the electrode pin (142) of the color electrode wire (140) is connectable; a microprocessor (160) installed in the case (110), configured to output a treatment start signal of either of urinary incontinence treatment and dysmenorrhea treatment whenever a treatment operation signal is input after a treatment mode selection signal of either of the urinary incontinence treatment mode and the dysmenorrhea treatment mode is input, and configured to output a treatment termination signal when a predetermined time elapses after the treatment start signal is output;
a treatment signal generator (170) configured to output a treatment signal to emit colored light having a predetermined wavelength range for urinary incontinence treatment or dysmenorrhea treatment when a treatment start signal of either of the urinary incontinence treatment and the dysmenorrhea treatment to turn on the LED (141a) of the color electrode (141) is input, and stop output of the treatment signal when the treatment termination signal is input; and
a power supply unit configured to supply driving power of the treatment mode selection switch (120), the treatment operation button (130), the color electrode (141), the microprocessor (160), and the treatment signal generator (170).

2. The portable device for use in treatment of urinary incontinence and dysmenorrhea using photon energy according to claim 1, wherein the power supply unit is a disposable battery or rechargeable battery (180) configured to supply the driving power of the treatment mode selection switch (120), the treatment operation button (130), the color electrode (141), the microprocessor (160), and the treatment signal generator (170), and the rechargeable battery (180) is adapted to receive charge power through a charge adapter insertable into and connectable to a charge jack (90) installed at one surface of the case (110).

3. The portable device for use in treatment of urinary incontinence and dysmenorrhea using photon energy according to claim 1, wherein the color electrode (141) comprises:
the LED (141a) configured to emit colored light having a predetermined wavelength range for urinary incontinence treatment or dysmenorrhea treatment;
an optical filter (141b) configured to filter, amplify and collect the colored light emitted from the LED (141a);
an electrode case (141c) having a hollow tubular shape and comprising a rear surface to which the color electrode wire (140) is connected, an inner space in which the LED (141a) and the optical filter (141b) are housed, and a front surface having a hole (141c1) through which the colored light passing through the optical filter (141b) is dischargeable to the outside, wherein one surface of a double-sided sticker (143) is attached to a periphery of the hole (141c1) of the front surface, and the other surface of the sticker (143) is attached to a urinary incontinence treatment area or a dysmenorrhea treatment area of the body.

4. The portable device for use in treatment of urinary incontinence and dysmenorrhea using photon energy according to claim 3, wherein the optical filter (141b) is formed of any one of quartz, crystal, and crystal glass, and has a transmission surface cut in a polygonal shape or a convex lens shape such that the colored light emitted from the LED (141a) is intensively radiatable to a urinary incontinence treatment area or a dysmenorrhea treatment area of the body through the hole (141c1) of the electrode case (141c).

5. The portable device for use in treatment of urinary incontinence and dysmenorrhea using photon energy according to claim 1, further comprising a speaker (161) configured to inform the user of treatment termination through an alarm sound or voice, or a vibrator (162) configured to inform the user of treatment termination through vibrations when the microprocessor (160) outputs a treatment termination signal.

6. The portable device for use in treatment of urinary incontinence and dysmenorrhea using photon energy according to claim 1, wherein a loop (111) to which a string is boundable to use the case (110) like a necklace, or a clip (112) that enables the case (110) to be worn on a waist belt, is formed at one surface of the case (110).

7. The portable device for use in treatment of urinary incontinence and dysmenorrhea using photon energy according to claim 1, comprising a wireless treatment mode selector (200) in wireless communication with the microprocessor (160), said wireless treatment mode selector (200) being configured to output a treatment mode selection signal of either of a urinary incontinence treatment mode and a dysmenorrhea treatment mode through a wireless communication unit (163) in a wireless manner to receive a treatment mode selection signal.

8. The portable device for use in treatment of urinary incontinence and dysmenorrhea using photon energy according to claim 7, wherein the wireless treatment mode selector (200) is any one of a personal computer (PC), a notebook, a smart phone, and a personal digital assistant (PDA), in which a program in wireless communication with the wireless communication unit (163) of the microprocessor (160) to transmit a treatment mode selection signal of either of the urinary incontinence treatment mode and the dysmenorrhea treatment mode through any one of radio frequency (RF), Bluetooth, and ZigBee types is installed, and providing a user interface (UI) screen that is able to transmit a treatment mode selection signal.

## Patentansprüche

1. Tragbare Vorrichtung zur Verwendung bei der Behandlung von Harninkontinenz und Dysmenorrhoe unter Verwendung von Photonenenergie, umfassend:
ein Gehäuse (110) mit einer hohlen röhrenartigen Form;
einen Behandlungsmodusauswahlschalter (120), der auf einer Oberfläche des Gehäuses (110) installiert und dazu konfiguriert ist, ein Behandlungsmodusauswahlsignal für eines aus einem Harninkontinenz-Behandlungsmodus und einem Dysmenorrhoe-Behandlungsmodus auszugeben;
eine Behandlungsbetriebstaste (130), die auf einer Oberfläche des Gehäuses (110) installiert und dazu konfiguriert ist, ein Behandlungsbetriebssignal auszugeben, sobald die Taste einmal gedrückt wird;
einen Farbelektrodendraht (140), an dessen einem Ende mindestens eine Farbelektrode (114) angeschlossen ist, umfassend eine lichtemittierende Diode (LED) (141a), die dazu konfiguriert ist, farbiges Licht mit einem vorgegebenen Wellenlängenbereich für die Harninkontinenzbehandlung oder Dysmenorrhoebehandlung zu emittieren, und an dessen anderem Ende ein Elektrodenstift (142) angeschlossen ist;
mindestens eine Elektrodensteckbuchse (150), die auf einer Oberfläche des Gehäuses (110) installiert ist, mit der der Elektrodenstift (142) des Elektrodendrahts (140) verbindbar ist;
einen Mikroprozessor (160) der in dem Gehäuse installiert und dazu konfiguriert ist, ein Behandlungsstartsignal für eines aus der Harninkontinenzbehandlung und der Dysmenorrhoebehandlung auszugeben, sobald ein Behandlungsbetriebssignal eingegeben wird, nachdem ein Behandlungsmodusauswahlsignal für eines aus dem Harninkontinenz-Behandlungsmodus und dem Dysmenorrhoe-Behandlungsmodus eingegeben wurde, und der dazu konfiguriert ist, ein Behandlungsbeendigungssignal auszugeben, wenn eine vorgegebene Zeit verstrichen ist, nachdem das Behandlungs-startsignal ausgegeben wurde;
einen Behandlungsignalgenerator (170), der dazu konfiguriert ist, ein Behandlungssignal zum Emittieren von farbigem Licht mit einem vorgegebenen Wellenlängenbereich für die Harninkontinenzbehandlung oder die Dysmenorrhoebehandlung zu emittieren, wenn ein Behandlungsstartsignal für eines aus der Harninkontinenzbehandlung und der Dysmenorrhoebehandlung zum Einschalten der LED (141a) der Farbelektrode (141) eingegeben wird, und die Ausgabe des Behandlungssignals zu stoppen, wenn das Behandlungsbeendigungssignal eingegeben wird; und
eine Stromversorgungseinheit, die dazu konfiguriert ist, Antriebsstrom für den Behandlungsmodusauswahlschalter (120), die Behandlungsbetriebstaste (130), die Farbelektrode (141), den Mikroprozessor (160) und den Behandlungssignalgenerator (170) zu liefern.

2. Tragbare Vorrichtung zur Verwendung bei der Behandlung von Harninkontinenz und Dysmenorrhoe unter Verwendung von Photonenenergie nach Anspruch 1, wobei die Stromversorgungseinheit eine Einwegbatterie oder eine wiederaufladbare Batterie (180) ist, die dazu konfiguriert ist, den Antriebsstrom für den Behandlungsmodusauswahlschalter (120), die Behandlungsbetriebstaste (130), die Farbelektrode (141), den Mikroprozessor (160) und den Behandlungssignalgenerator (170) zu liefern, und die wiederaufladbare Batterie (180) dazu eingerichtet ist, Ladestrom durch einen Ladeadapter zu erhalten, der in eine Ladesteckbuchse einsetzbar und damit verbindbar ist, die auf einer Oberfläche des Gehäuses (110) installiert ist.

3. Tragbare Vorrichtung zur Verwendung bei der Behandlung von Harninkontinenz und Dysmenorrhoe unter Verwendung von Photonenenergie nach Anspruch 1, wobei die Farbelektrode (141) umfasst:
die LED (141a), die zum Emittieren von farbigem Licht mit einem vorgegebenen Wellenlängenbereich für die Harninkontinenzbehandlung oder Dysmenorrhoebehandlung konfiguriert ist;
einen optischen Filter (141b), der dazu konfiguriert ist, das von der LED (141a) emittierte farbige Licht zu filtern, zu verstärken und zu sammeln;
ein Elektrodengehäuse (141c) mit einer hohlen röhrenartigen Form, das eine Rückseite umfasst, an der der Farbelektrodendraht (140) angeschlossen ist, einen Innenraum, in dem die LED (141a) und der optische Filter (141b) angeordnet sind, und eine Vorderseite mit einer Öffnung (141c1), durch die das durch den optischen Filter (141b) verlaufende farbige Licht nach außen abgegeben werden kann, wobei eine Seite eines doppelseitigen Aufklebers (143) an einem Umfang der Öffnung (141c1) der Vorderseite befestigt ist, und die andere Seite des Aufklebers (143) an einem Harninkontinenzbehandlungsbereich oder einem Dysmenorrhoebehandlungsbereich des Körpers befestigt wird.

4. Tragbare Vorrichtung zur Verwendung bei der Behandlung von Harninkontinenz und Dysmenorrhoe unter Verwendung von Photonenenergie nach Anspruch 3, wobei der optische Filter (141b) aus einem aus Quarz, Kristall und Kristallglas besteht und eine Durchtrittsfläche aufweist, die zu einer polygonalen Form oder einer konvexen Linsenform geschnitten ist, so dass das von der LED (141a) emittierte farbige Licht intensiv auf einen Harninkontinenzbehandlungsbereich oder einen Dysmenorrhoebehandlungsbereich des Körpers durch die Öffnung (141c1) des Elektrodengehäuses (141c) abstrahlbar ist.

5. Tragbare Vorrichtung zur Verwendung bei der Behandlung von Harninkontinenz und Dysmenorrhoe unter Verwendung von Photonenenergie nach Anspruch 1, des Weiteren umfassend einen Lautsprecher (161), der dazu konfiguriert ist, den Benutzer über die Behandlungsbeendigung durch einen akustischen Alarm oder eine Stimme zu informieren, oder einen Vibrator (162), der dazu konfiguriert ist, den Benutzer über die Behandlungsbeendigung durch Vibrationen zu informieren, wenn der Mikroprozessor (160) ein Behandlungsbeendigungssignal ausgibt.

6. Tragbare Vorrichtung zur Verwendung bei der Behandlung von Harninkontinenz und Dysmenorrhoe unter Verwendung von Photonenenergie nach Anspruch 1, wobei eine Schlaufe (111), an die eine Schnur bindbar ist, um das Gehäuse (110) wie eine Kette zu tragen, oder ein Clip (112), der das Tragen des Gehäuses (110) an einem Gürtel ermöglicht, auf einer Oberfläche des Gehäuses (110) ausgebildet ist

7. Tragbare Vorrichtung zur Verwendung bei der Behandlung von Harninkontinenz und Dysmenorrhoe unter Verwendung von Photonenenergie nach Anspruch 1, umfassend einen drahtlosen Behandlungsmodusselektor (200), der in drahtloser Kommunikation mit dem Mikroprozessor (160) steht, wobei der drahtlose Behandlungsmodusselektor (200) dazu konfiguriert ist, ein Behandlungsmodusauswahlsignal für eines aus einem Harninkontinenz-Behandlungsmodus und einem Dysmenorrhoe-Behandlungsmodus durch eine drahtlose Kommunikationseinheit (163) auf drahtlose Weise auszugeben, um ein Behandlungsmodusauswahlsignal zu empfangen.

8. Tragbare Vorrichtung zur Verwendung bei der Behandlung von Harninkontinenz und Dysmenorrhoe unter Verwendung von Photonenenergie nach Anspruch 7, wobei der drahtlose Behandlungsmodus (200) eines aus einem Personal Computer (PC), einem Notebook, einem Smartphone und einem persönlichen digitalen Assistenten (PDA) ist, in dem ein Programm in drahtloser Kommunikation mit der drahtlosen Kommunikationseinheit (163) des Mikroprozessors (160) zur Übertragung eines Behandlungsmodusauswahlsignals für eines aus dem Harninkontinenz-Behandlungsmodus und dem Dysmenorrhoe-Behandlungsmodus durch eines aus Funkfrequenz (RF), Bluetooth und ZigBee installiert ist, und zum Bereitstellen eines Benutzerschnittstellenbildschirms (UE), der dazu in der Lage ist, ein Behandlungsmodusauswahlsignal zu übertragen

## Revendications

1. Dispositif portable destiné à une utilisation dans le traitement de l'incontinence urinaire et de la dysménorrhée en utilisant l'énergie photonique, comprenant :
un boîtier (110) ayant une forme tubulaire creuse ;
un commutateur de sélection de mode de traitement (120) installé au niveau d'une surface du boîtier (110) et configuré pour émettre un signal de sélection de mode de traitement de l'un ou l'autre du mode de traitement de l'incontinence urinaire et du mode de traitement de la dysménorrhée ;
un bouton de fonctionnement de traitement (130) installé au niveau d'une surface du boîtier (110) et configuré pour émettre un signal de fonctionnement de traitement quel que soit le bouton poussé une fois ;
un fil d'électrode de couleur (140) ayant une extrémité au niveau de laquelle au moins une électrode de couleur (141) comprenant une diode électroluminescente (DEL) (141a) configurée pour émettre de la lumière colorée ayant une plage de longueurs d'onde prédéterminée pour le traitement de l'incontinence urinaire ou le traitement de la dysménorrhée est connectée, et une autre extrémité au niveau de laquelle une broche d'électrode (142) est connectée;
au moins une prise de l'électrode (150) installée au niveau d'une surface du boîtier (110), au niveau de laquelle la broche d'électrode (142) du fil d'électrode de couleur (140) peut être connectée ;
un microprocesseur (160) installé dans le boîtier (110), configuré pour émettre un signal de démarrage de traitement de l'un ou l'autre du traitement de l'incontinence urinaire et du traitement de la dysménorrhée quel que soit le signal de fonctionnement de traitement saisi après un signal de sélection de mode de traitement de l'un ou l'autre du mode de traitement de l'incontinence urinaire et du mode de traitement de la dysménorrhée qui est saisi, et configuré pour émettre un signal de fin de traitement lorsqu'une durée prédéterminée s'écoule après que le signal de démarrage de traitement soit émis ;
un générateur de signal de traitement (170) configuré pour émettre un signal de traitement afin d'émettre une lumière colorée ayant une plage de longueurs d'onde prédéterminée pour le traitement de l'incontinence urinaire ou le traitement de la dysménorrhée lorsqu'un signal de démarrage de traitement de l'un ou l'autre du traitement de l'incontinence urinaire et du traitement de la dysménorrhée afin d'allumer la DEL (141a) de l'électrode de couleur (141) est saisi, et arrêter l'émission du signal de traitement lorsque le signal de fin de traitement est saisi ; et
une unité d'alimentation d'énergie configurée pour alimenter l'énergie d'entraînement du commutateur de sélection de mode de traitement (120), du bouton de fonctionnement de traitement (130), de l'électrode de couleur (141), du microprocesseur (160), et du générateur de signal de traitement (170).

2. Dispositif portable destiné à une utilisation dans le traitement de l'incontinence urinaire et de la dysménorrhée en utilisant l'énergie photonique selon la revendication 1, l'unité d'alimentation d'énergie étant une batterie jetable ou une batterie rechargeable (180) configurée pour alimenter l'énergie d'entraînement du commutateur de sélection de mode de traitement (120), du bouton de fonctionnement de traitement (130), de l'électrode de couleur (141), du microprocesseur (160), et du générateur de signal de traitement (170), et la batterie rechargeable (180) étant adaptée pour recevoir l'énergie de charge à travers un adaptateur de charge pouvant être inséré dans, et connecté à, une prise de charge (90) installée au niveau d'une surface du boîtier (110).

3. Dispositif portable destiné à une utilisation dans le traitement de l'incontinence urinaire et de la dysménorrhée en utilisant l'énergie photonique selon la revendication 1, l'électrode de couleur (141) comprenant :
la DEL (141a) configurée pour émettre de la lumière colorée ayant une plage de longueurs d'onde prédéterminée destinée au traitement de l'incontinence urinaire ou au traitement de la dysménorrhée ;
un filtre optique (141b) configuré pour filtrer, amplifier et recueillir la lumière colorée émise depuis la DEL (141a) ;
un boîtier d'électrode (141c) ayant une forme tubulaire creuse et comprenant une surface dorsale au niveau de laquelle le fil d'électrode de couleur (140) est connecté, un espace interne dans lequel la DEL (141a) et le filtre optique (141b) sont logés, et une surface avant présentant un trou (141c1) à travers lequel la lumière colorée passant à travers le filtre optique (141b) peut être déchargée vers l'extérieur, où une surface d'un élément collant double face (143) est fixée à une périphérie du trou (141c1) de la surface avant, et l'autre surface de l'élément collant (143) est fixée à une zone de traitement de l'incontinence urinaire ou une zone de traitement de la dysménorrhée du corps.

4. Dispositif portable destiné à une utilisation dans le traitement de l'incontinence urinaire et de la dysménorrhée en utilisant l'énergie photonique selon la revendication 3, où le filtre optique (141b) est formé à partir de n'importe lequel parmi le quartz, le cristal, et le verre cristallin, et présente une découpe de surface de transmission selon une forme polygonale ou une forme de lentille convexe de sorte que la lumière colorée émise depuis la DEL (141a) puisse intensément être rayonnante vers une zone de traitement de l'incontinence urinaire ou une zone de traitement de la dysménorrhée du corps à travers le trou (141c1) du boîtier d'électrode (141c).

5. Dispositif portable destiné à une utilisation dans le traitement de l'incontinence urinaire et de la dysménorrhée en utilisant l'énergie photonique selon la revendication 1, comprenant en outre un haut-parleur (161) configuré pour informer l'utilisateur de l'achèvement du traitement à travers un son ou une voix d'alarme, ou un vibrateur (162) configuré pour informer l'utilisateur de l'achèvement du traitement à travers des vibrations lorsque le microprocesseur (160) émet un signal de fin de traitement.

6. Dispositif portable destiné à une utilisation dans le traitement de l'incontinence urinaire et de la dysménorrhée en utilisant l'énergie photonique selon la revendication 1, dans lequel une boucle (111) au niveau de laquelle un fil peut être lié pour l'utilisation du boîtier (110) comme un collier, ou une attache (112) qui permet au boîtier (110) d'être porté à la taille, est formée au niveau d'une surface du boîtier (110).

7. Dispositif portable destiné à une utilisation dans le traitement de l'incontinence urinaire et de la dysménorrhée en utilisant l'énergie photonique selon la revendication 1, comprenant un sélecteur de mode de traitement sans fil (200) en communication sans fil avec le microprocesseur (160), ledit sélecteur de mode de traitement sans fil (200) étant configuré pour émettre un signal de sélection de mode de traitement de l'un ou l'autre d'un mode de traitement de l'incontinence urinaire et d'un mode de traitement de la dysménorrhée à travers une unité de communication sans fil (163) d'une manière sans fil pour recevoir un signal de sélection de mode de traitement.

8. Dispositif portable destiné à une utilisation dans le traitement de l'incontinence urinaire et de la dysménorrhée en utilisant l'énergie photonique selon la revendication 7, dans lequel le sélecteur de mode de traitement sans fil (200) est n'importe lequel d'un ordinateur personnel (PC), d'un ordinateur portable, d'un téléphone intelligent, et d'un assistant numérique personnel (PDA), dans lequel un programme en communication sans fil avec l'unité de communication sans fil (163) du microprocesseur (160) pour transmettre un signal de sélection de mode de traitement de l'un ou l'autre du mode de traitement de l'incontinence urinaire et du mode de traitement de la dysménorrhée à travers n'importe quels types parmi la radio fréquence (RF), le bluetooth, et le ZigBee est installé, et fournissant un écran d'interface utilisateur (UI) qui est capable de transmettre un signal de sélection de mode de traitement.
